# EUROPÄISCHE PATENTSCHRIFT

(11) **EP 2 222 330 B1**
(45) Veröffentlichungstag und Bekanntmachung des Hinweises auf die Patenterteilung: **11.10.2017**
(21) Anmeldenummer: 08863153.6
(22) Anmeldetag: 18.12.2008
(51) Int. Cl.: A61K 38/48, A61P 31/00

(54) **BEHANDLUNG INFLAMMATORISCHER KRANKHEITEN MIT ACE2**
TREATMENT OF INFLAMMATORY ILLNESSES WITH ACE2
TRAITEMENT DE MALADIES INFLAMMATOIRES PAR ACE2

(30) Priorität: 18.12.2007 AT 20582007
(43) Veröffentlichungstag der Anmeldung: 01.09.2010
(60) Teilanmeldung: 17178557.9
(73) Patentinhaber: Apeiron Biologics AG, 1030 Wien (AT)
(72) Erfinder: LOIBNER, Hans, A-1230 Wien (AT); SCHUSTER, Manfred, A-2191Schrick (AT)
(74) Vertreter: Sonn & Partner Patentanwälte
(86) Internationale Anmeldenummer: PCT/AT2008/000460
(87) Internationale Veröffentlichungsnummer: WO 2009/076694

(56) Entgegenhaltungen:
- EP-A- 1 723 962
- WO-A-02/39997
- WO-A-2004/000367
- WO-A1-02/098448
- IMAI Y ET AL: "Angiotensin-converting enzyme 2 protects from severe acute lung failure" NATURE, NATURE PUBLISHING GROUP, LONDON, UK, Bd. 436, Nr. 7047, 7. Juli 2005 (2005-07-07), Seiten 112-116, XP002349987 ISSN: 0028-0836
- JUN CHEN AND KANTA SUBBARAO: "The Immunobiology of SARS" ANNU. REV. IMMUNOL., Bd. 25, 2007, Seiten 443-472, XP002521974
- RICHARDSON MARK A ET AL: "Treatment of sepsis-induced acquired protein C deficiency reverses angiotensin-converting enzyme-2 inhibition and decreases pulmonary inflammatory response" THE JOURNAL OF PHARMACOLOGY AND EXPERIMENTAL THERAPEUTICS, Bd. 325, Nr. 1, Januar 2008 (2008-01), Seiten 17-26, XP009114738

## Beschreibung

Die vorliegende Erfindung betrifft das Gebiet der Behandlung inflammatorischer Krankheiten.

Eine inflammatorische Reaktion ist ein Prozess, bei dem sich Abwehrzellen auf den Weg zu einer Infektionsquelle machen und dort die Beseitigung der Ursache sicherstellen. Unterschiedliche Mittlerstoffe werden hierbei freigesetzt, die zu der Beseitigung beitragen, aber auch die Entzündungssymtome erzeugen. Bei Fehlregulierungen der Reaktion können diese Symptome den haupt sächlichen Schaden verursachen bzw. generell die Krankheitsquelle sein (z.B. bei Allergien). Eine Unterscheidung kann auch zwischen akuten Inflammationen (wie Sepsis) und latenten chronischen Inflammationen (wie beispielsweise Rheumatismus) gemacht werden. Inflammationen können auch künstlich verursacht werden, z.B. bei Organtransplantationen, die letztendlich zur Abstoßung des Fremdorgans führen können. Ebenso können Inflammationen als Nebenwirkung durch bestimmte Medikamente hervorgerufen werden.

Bei all diesen Zuständen kann entweder zur hauptsächlichen Behandlung oder auch nur zur Symptomlinderung eine künstliche Regulierung der Immunantwort sinnvoll sein.

Während einer Inflammation sind eine Reihe von Zytokinen maßgeblich an dem Fortschreiten einer Immunreaktion beteiligt. Aktivierte CD4-T-Zellen produzieren Interleukin-2, das für die Aktivierung sowohl der CD8-T-Zellen als auch der B-Zellen wesentlich ist. Weiters produzieren CD4-T-Zellen weitere Zytokine, wie IFN-gamma, welches die Makrophagenaktivität verstärkt. TNF-alpha regelt die Aktivität verschiedener Immunzellen und kann den Zelltod, Zellproliferation, Zelldifferenzierung und Ausschüttung weiterer Zytokine anregen. Insbesondere trägt er zu Symptomen wie Fieber auslösend bei.

Es ist ein Ziel der vorliegende Erfindung einen Regulator des Immunsystems zur Verfügung zu stellen, insbesondere zur Behandlung von Inflammationen (Entzündungen).

Die Erfindung betrifft daher ein Protein oder eine Nukleinsäure, welche das Protein kodiert, wobei das Protein ACE2 ist, zur therapeutischen Behandlung oder Vorbeugung einer Inflammation (bzw. inflammatorischen Krankheit). Ebenso betrifft die vorliegende Erfindung die Verwendung von einem ACE2 Protein oder einer ACE2 kodierenden Nukleinsäure zur Herstellung einer pharmazeutischen Zusammensetzung zur Behandlung oder Vorbeugung einer Inflammation. Gleichfalls wird die Verwendung von ACE2 Protein oder einer ACE2 kodierenden Nukleinsäure zur Immunmodulation in einem Patienten, wie der Behandlung oder Vorbeugung einer Inflammation, vorgesehen.

Angiotensin Converting Enzyme 2 (ACE2) ist ein essentielles Enzym des Renin-Angiotensin-Aldosteron Systems, welches als membranverankertes Glykoprotein auf diversen Organen, wie Herz, Niere, Leber und Lunge, aber auch auf Blutgefäßen exprimiert wird.

ACE2 wurde 1997 als ACE homologes Enzym entdeckt (Genbank Acc.: BAB40370, kodiert durch eine Nukleinsäure mit der Sequenz nach Genbank Acc.: AB046569). Anfänglich wurde ihm dieselbe enzymatische Aktivität wie ACE zugerechnet (US 6,989,363). Erst später wurde entdeckt, dass ACE2 eine gänzlich andere, sogar antagonistische Wirkungseise zu ACE aufweist (WO 2004/000367). ACE2 ist eine Carboxypeptidase, die zahlreiche Peptidsubstrate mit stark unterschiedlicher Selektivität und Aktivität spaltet. ACE2 ist auch ein zellulärer Bindungspartner von SARS-Coronaviren. Die Herunterregulierung von ACE2 oder die Verabreichung von ACE2, um Virus-Rezeptoren zu blockieren, kann daher die Suszeptibilität ACE2 präsentierender Zellen mindern (WO 2006/122819, Lang et al., Virology (2006) 353(2): 474, abstract). Die beschriebenen Funktionen für ACE2 sind vor allem die Umwandlung von Ang II zu Ang 1-7, wobei Substrat und Produkt dieser Reaktion antagonistische Eigenschaften aufweisen. Ang II wirkt im Wesentlichen vasokonstriktiv und blutdrucksteigernd. Ang 1-7 wirkt vasodilatorisch und weist bei Nieren-, Lungen- und Herzerkrankungen einen Schutzeffekt auf (WO 2004/000367).

Das ACE2 Produkt Ang 1-7 hemmt im Weiteren ACE, das Enzym, welches für die Produktion von Ang II maßgeblich verantwortlich ist. Die Expression von ACE2 wird durch diverse Stimuli gesteuert. Es wurde nun gefunden, dass ACE2 durch das Auftreten inflammatorischer Zytokine, wie TNF-alpha, IFN-gamma oder IL-4, herunterreguliert wird, was in weiterer Folge zu diversen Erkrankungen und zu einer Akkumulation von Ang II in den betroffenen Kompartimenten und zu einer Potenzierung der eingeleiteten Immunantwort führt. Zytokine dienen im Wesentlichen der Kommunikation diverser Zelltypen des Immunsystems. Üblicherweise besteht einer der ersten Schritte einer naszierenden Inflammation darin, dass antigene Substanzen durch Antigen Präsentierende Zellen (APCs) aufgenommen werden und als fremd eingestuft werden. In weiterer Folge kommt es zu einem ersten Ausstoß inflammatorischer Zytokine durch die betroffenen APCs, welche dadurch weitere Zellen des Immunsystems alarmieren. Dieser Mechanismus ist hoch reguliert und kontrolliert, um eine Immunantwort nur dann einzuleiten, wenn diese auch tatsächlich berechtigt ist, und diese wieder abzudrehen, wenn die antigene Substanz neutralisiert wurde. Dennoch kann es vorkommen, dass diese eingeleitete Immunantwort außer Kontrolle gerät und sich gegen den eigenen Organismus wendet. Die Akkumulation von Ang II, z.B in diversen Nieren-, Herz- und Lungenerkrankungen, bedingt eine fortschreitende Inflammation und auch eine gesteigerte Infiltration des betroffenen Gewebes durch Zellen des Immunsystems und in weiterer Folge ein Überschießen der Immunantwort. Ein Beispiel ist die Sepsis, wo sehr große Mengen inflammatorischer Zytokine ausgeschüttet werden und systemisch eine ausufernde Immunantwort eingeleitet wird, was zu einer massiven Schädigung so gut wie aller Organe führt. Weiters kann auch ein allergischer Schub oder Ausbruch einer Autoimmunerkrankung behandelt oder vorgebeugt werden. Eine Schlüsselstelle ist hierbei allerdings immer die zelluläre Immunantwort als Reaktion auf einen Stimulus, welche in einer sich potenzierenden Amplifikationskaskade den primären Zweck, eine fremde Substanz zu neutralisieren, bei weitem übererfüllt und in weiterer Folge dem Organismus schädigt.

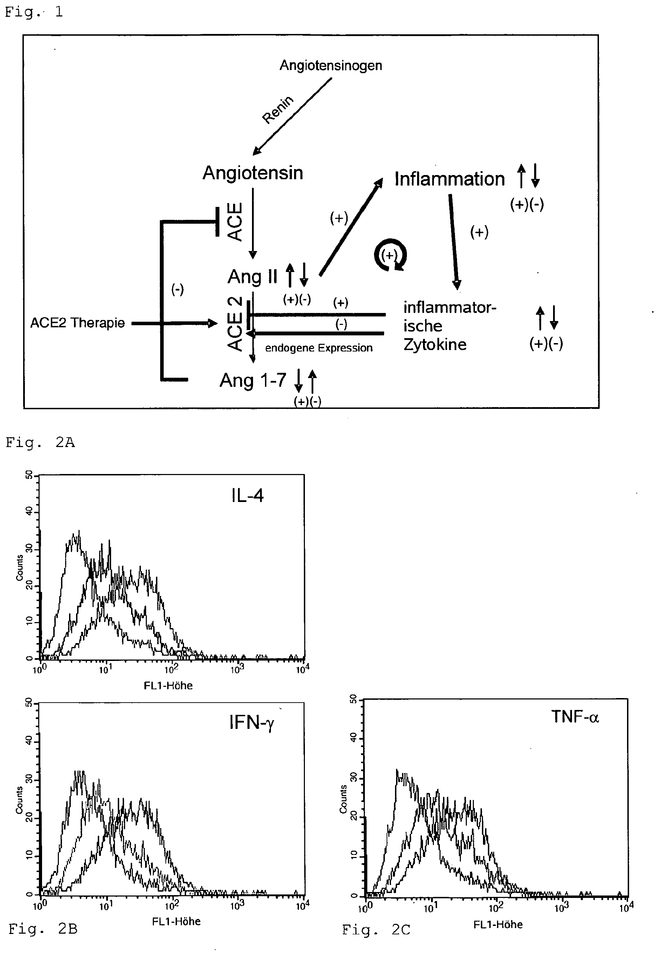

Der erste Schritt der aufkeimenden Immunantwort ist die Aussendung inflammatorischer Signale in Form von Zytokinen. Wesentliche Vertreter hierfür sind zum Beispiel IL-4, IFN-gamma oder TNF-alpha. Substanzen, die die Eigenschaft haben, diese Zytokinexpression nach Stimulierung der Immunzelle zu unterbinden oder abzuschwächen, sind brauchbare Therapeutika zur Attenuierung einer überschießenden Immunantwort. Die ACE2 Expression nimmt in Gegenwart inflammatorischer Zytokine auf zellulärerer Ebene stark ab, was zu einer Potenzierung der Inflammation vor allem durch Akkumulation von Ang II, durch die Abnahme von Ang 1-7 und durch das dadurch bedingte Ausbleiben einer Herabsetzung der Ang II Nachbildung führt (Fig. 1). Die deswegen stark zunehmenden Ang II Konzentrationen fördern aufgrund der stark inflammatorischen Eigenschaften von Ang II die weitere Potenzierung der Inflammation, welche in weiterer Folge zu einer noch deutlicheren Attenuierung der ACE2 Expression führt. Um diesem Teufelskreis zu entkommen, wird erfindungsgemäß ACE2 therapeutisch verabreicht und somit einer Ang II Akkumulation vorgebeugt und dabei die Inflammation gedämpft: ACE2 verringert unmittelbar die hohen Ang II Titer, was die durch Ang II sich laufend steigernde Inflammation abschwächt. Ang 1-7 wird nachgebildet und schwächt durch dessen anti-inflammatorische Wirkung ebenfalls die Inflammation ab. Weiters limitiert Ang 1-7 durch dessen Eigenschaft, ACE zu inhibieren, die Nachproduktion von Ang II. Das Abflauen der Inflammation bewirkt, dass die ausgeschütteten Zytokine auf ein Normalmaß zurückkehren, und dass es wieder zu einer endogenen ACE2 Expression kommt, die nachhaltig für den Abbau von Ang II und die Entstehung von Ang 1-7 sorgt und wieder zu einem stabilen funktionellen RAS führt. In weiterer Folge stellt sich wieder ein sich selbst regulierendes beständiges Gleichgewicht der agierenden Komponenten des RAS ein. Eine erneute ACE2 Gabe kann somit gänzlich ausbleiben, wenn der ursprüngliche Stimulus des Immunsystems neutralisiert wurde. Eine schematische Darstellung der erwähnten Mechanismen ist in Fig. 1 wiedergegeben. Durch Gabe von ACE2 wird ein Ausweg aus der sich potenzierenden Inflammation geschaffen.

Vorzugsweise ist die Inflammation eine lokale Inflammation eines Gewebes oder Organs und/oder eine systemische Inflammation. Des Weiteren ist, basierend auf dem allgemeinen Mechanismus, sowohl die Behandlung von chronischen als auch akuten Inflammationen möglich. Insbesondere kann die Inflammation Rheumatitis, Sepsis, Arthritis, rheumatoiden Systemischen Lupus Erythematodes, oder Sklerodermie umfassen. Sie kann durch mechanische oder chemische Zell- oder Gewebeschäden oder Wunden, Infektionen, insbesondere von Pathogenen wie Viren, Bakterien oder Pilzen, durch Implantate, inklusive Organimplantate, sowie Medikamente hervorgerufen werden.

Vorzugsweise wird das rekombinante ACE2 als ACE2-Protein eingesetzt. ACE2 Sequenzen sind hinreichend bekannt, und es kann problemlos durch Einbringung von geeigneten Vektoren, welche ACE2 kodieren, in Expressionssystemen, insbesondere eukaryotischen, produziert werden. Solche Systeme sind z.B. Säugerzelllinien wie CHO (Chinese Hamster Ovary) Zellen und NS0 Mauszellen oder Insektenzellen, z.B. Sf9. Zur Expression kann ein solcher Vektor bestimmte Zell-spezifische oder allgemeine Promotoren aufweisen.

Vorzugsweise ist das Protein (für das auch die ACE2 Nukleinsäure kodiert) wasserlösliches ACE2, insbesondere ohne Membrandomäne. Die menschliche ACE2 Sequenz ist durch die SEQ ID NO:1 angegeben:

Dabei wird die autologe Signalsequenz (unterstrichen) durch die Wirtszelle für die Ausschleusung abgespalten. Vorzugsweise umfasst daher das erfindungsgemäße ACE2 Protein eine ACE2 Sequenz entsprechend der SEQ ID NO: 1 ab der Position 18. In weiteren Ausführungsformen weist das ACE2 Polypeptid keine transmembrane Domäne auf. Diese Transmembrandomäne befindet sich am C-Terminus der SEQ ID NO:1. Es handelt sich daher dann um lösliches ACE2. Besonders bevorzugte Ausführungsformen umfassen dabei lösliche ACE2-Polypeptide, deren Polypeptidkette aus den Aminosäuren die SEQ ID NO:1 bis zur Aminosäureposition 740 umfassen, oder enzymatisch aktive Fragmenten davon. Ein weiteres lösliches ACE2 Protein besteht aus den Aminosäuren 18-615 der SEQ ID NO: 1.

Die Löslichkeit eines Proteins wird nicht nur durch seine Aminosäuresequenz, sondern auch durch seine Faltung sowie durch post-translationelle Modifikationen bestimmt. Es sind vor allem geladene Zuckerstrukturen, die maßgeblich die Löslichkeit eines Proteins steigern und dessen pharmakologisches Profil beeinflussen. Der lösliche Abschnitt von ACE2 enthält 7 N-Glykosylierungsstellen. Vorzugsweise sind mindestens 80% der möglichen N-Glykosylierungspositionen glykosyliert und/oder das ACE2 Protein weist einen Zuckeranteil von größer als 10% (Massen-% des gesamten ACE2) oder 11%, 12%, 13%, 14%, vorzugsweise größer als 15% oder 16%, 17%, 18%, 19%, insbesondere größer als 20 % oder 21%, 22%, 23% 24% oder 25%, auf.

Obwohl menschliches ACE2 für die meisten Ausführungsformen bevorzugt ist, wird auch ACE2 von Maus, Ratte, Hamster, Schwein, Primaten oder Rind ermöglicht. ACE2 ist ein universelles Enzym in allen Säugetieren mit dem identischen Substrat Ang II. Daher ist es auch in Fremdorganismen einsetzbar. Somit können mit dem erfindungsgmäßen Protein (oder seiner Nukleinsäure) unabhängig vom Ursprung von ACE2 z.B. Menschen, Mäuse, Ratten, Hamster, Schweine, Primaten oder Rinder behandelt werden.

Erfindungsgemäß kann eine pharmazeutische Zusammenfassung umfassend das ACE2 Protein oder eine ACE2 kodierende Nukleinsäure zur Verfügung gestellt werden. Solche Zusammensetzungen können pharmazeutisch geeignete Salze derselben, zusätzlich Puffer, Tonizitätskomponenten oder pharmazeutisch geeignete Träger umfassen. Insbesondere ACE2 Nukleinsäure können in geeigneten therapeutischen Vektorsystemen vorgesehen werden. Pharmazeutische Träger-Substanzen dienen der besseren Verträglichkeit der Zusammensetzung und ermöglichen bessere Löslichkeit sowie bessere Bioverfügbarkeit der Wirksubstanzen. Beispiele hierfür sind Emulgatoren, Verdickungsmittel, Redoxkomponenten, Stärke, Alkohollösungen, Polyethylenglycol oder Lipide. Die Auswahl eines geeigneten pharmazeutischen Trägers hängt stark von der Art der Verabreichung ab. Für orale Verabreichungen können flüssige oder feste Träger verwendet werden, für Injektionen sind flüssige Endzusammensetzungen erforderlich.

Vorzugsweise umfasst das erfindungsgemäß zu verwendende Medikament Puffersubstanzen oder tonische Substanzen. Mittels Puffer kann der pH-Wert des Medikaments auf physiologische Konditionen eingestellt werden und weiters können pH-Schwankungen abgeschwächt bzw. gepuffert werden. Ein Beispiel hierfür ist ein Phosphatpuffer. Tonische Substanzen dienen zur Einstellung der Osmolarität und können ionische Substanzen, wie zum Beispiel anorganische Salze, wie NaCl, oder auch nicht-ionische Substanzen, wie zum Beispiel Glycerin oder Kohlenhydrate, umfassen.

Bevorzugterweise ist die erfindungsgemäß zu verwendende Zusammensetzung zur systemischen, topischen, oralen oder intranasalen Verabreichung geeignet hergerichtet. Diese Verabreichungsformen des Medikaments der vorliegenden Erfindung ermöglichen eine schnelle und unkomplizierte Aufnahme. Zur oralen Verabreichung können beispielsweise feste bzw. flüssige Medikamente direkt oder aufgelöst bzw. verdünnt eingenommen werden.

Das erfindungsgemäß zu verwendende Medikament ist vorzugsweise zur intravenösen, intraarteriellen, intramuskulären, intravaskulären, intraperitonealen oder subkutanen Verabreichung geeignet hergerichtet. Hierfür eignen sich beispielsweise Injektionen oder Transfusionen. Verabreichungen direkt in die Blutbahn haben den Vorteil, dass die Wirkstoffe des Medikaments im gesamten Körper verteilt werden und die Zielgewebe schnell erreichen.

Die vorliegende Erfindung wird durch die folgenden Figuren und Beispiele illustriert Figuren:
Fig. 1: Schematische Darstellung der Wiederherstellung eines funktionellen RAS durch ACE2 Therapie. Rote (+) Pfeile stellen Effekte der ausufernden Immunreaktivität dar während blaue (-) Pfeile Änderungen bedingt durch die ACE2 Therapie bezeichnen.
Fig. 2: ACE2 spezifische FACS Analytik von Vero E6 Zellpräparationen nach 48 stündiger Inkubation mit 10 ng/ml IL-4 (A), IFN-gamma (B) oder TNF-alpha (C) (Kurven mit mittlerem Peak) im Vergleich zu einer unstimulierten Kotrollgruppe (rote Kurven mit Peak rechts) und einer Kontrollserie (schwarze Kurven mit Peak links).
Fig. 3: Messung von TNF-alpha in PBMC Kulturüberständen 16 Stunden nach Stimulation mit LPS, PHA und LPS + PHA, ohne (schwarze Balken, links) oder in Gegenwart von ACE2 (graue Balken, mitte) oder ACE2 und Ang II (blaue Balken, rechts).
Fig. 4: Gemessene Ang II Konzentrationen in einem LPS induzierten Sepsismodell in Schweinen: blaue Kurve: APN 01 (rACE2) behandelte Tiere, graue Kurve: Placebo behandelte Tiere, graue Kurve (schwarze Punkte): gesunde Tiere nach APN 01 Administration.
Fig. 5: Gemessene ACE2 Aktivität in Mäusen, Schweinen und Rhesus Makaken.
Fig. 6: TNF-alpha Serumkonzentration in einem LPS induzierten Sepsismodell in Schweinen. ACE2 behandelte Tiere wurden in Blau, Placebo behandelte Tiere in Grau dargestellt. TNF- alpha Konzentrationen wurden auf die jeweiligen Ausgangswerte zu Therapiebeginn (100%) normiert.
Fig. 7: TNF- alpha Serumkonzentration in einem durch mekoniumaspirationinduzierten ARDS Modell in Schweinen. ACE2 behandelte Tiere wurden in Blau, Placebo behandelte Tiere in Grau dargestellt.

### Beispiele:

### Beispiel 1: Verlust der ACE2 Expression in Gegenwart inflammatorischer Zytokine

Die Nieren Zelllinie (Ceropithecus aethiops) Vero E6 exprimiert unter den üblichen Kulturbedingungen ACE2 als membranverankertes Glykoprotein. Vero E6 wurden für 48 Stunden mit 10 ng/ml IL-4, IFN-gamma oder TNF-alpha inkubiert und Änderungen hinsichtlich der ACE2 Oberflächenexpression mittels FACS Analytik unter Verwendung eines polyklonalen ACE2 spezifischen Ziegenantikörpers und eines ziegenspezifischen FITC markierten Antikörpers analysiert. In Fig. 2 sind die jeweiligen Histogramme dargestellt. Die zugehörige Auswertung ist in Tabelle 1 zusammengefaßt. Durch Inkubation mit IL-4, IFN-gamma oder TNF-alpha wurde die ACE2 Expression deutlich herabgesetzt. Während eine ACE2 Positivität von 51±3% in nicht stimulierten Zellen gemessen wurde, war diese auf 28±2, 22±1 und 39±2% jeweils nach IL-4, INF-gamma und TNF-alpha Stimulation herabgesetzt (Fig. 2).

**Tabelle 1: ACE2 spezifische FACS Analytik gemessen nach Inkubation von Vero E6 nach 48 stündiger Inkubation mit 10 ng/ml IL-4, IFN-gamma oder TNF-alpha im Vergleich zu einer unstimulierten Kontrollgruppe.**

| Stimulation | IL-4 | IFN-gamma | TNF-alpha | 0̸ |
|---|---|---|---|---|
| Positivität | 28±3 | 22±1 | 39±2 | 51±3 |
| Negativkontrolle | 5 | 2 | 4 | 6 |

### Beispiel 2: Attenuierung der Immunreaktivität von PBMCs

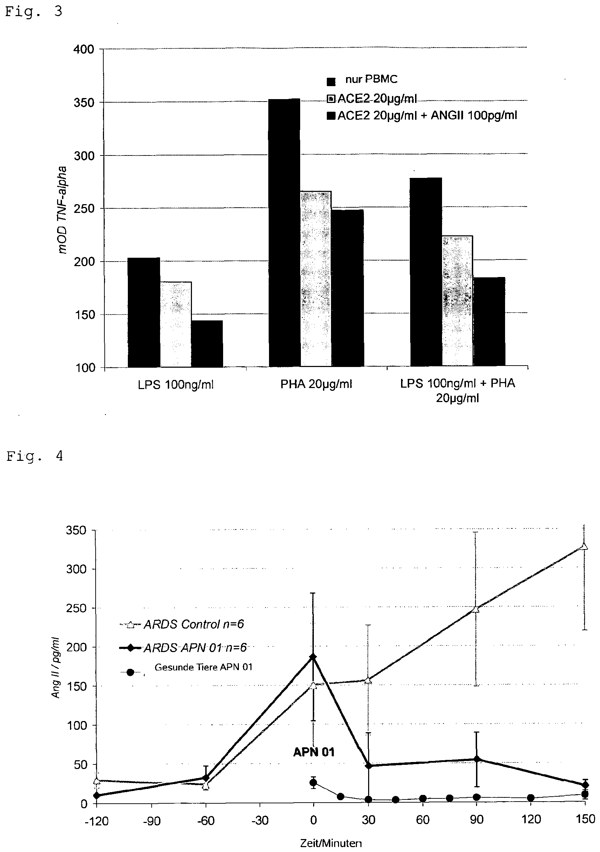

In diesem Beispiel wird der Effekt von ACE2 auf die Zytokinexpression stimulierter PBMCs (Periphere Mononukleare Blut Zellen) dargelegt. Es wurde eine PBMC Präparation und somit das gesamte Lymphozytenspektrum des Spenders im Ansatz verwendet, um das Zusammenspiel unterschiedlicher Lymphozyten zu ermöglichen. Einem gesunden Spender wurde Vollblut abgenommen und die darin enthaltenen PBMCs durch Zentrifugation abgetrennt. Diese Zellen wurden in weiterer Folge mit stark immunogenen Substanzen, wie Lipo Poly Saccharid (LPS, 100 ng/ml) und Phyto Hämagglutinin (PHA, 20 µg/ml), und einer Kombination beider Substanzen in Gegenwart von Ang II, ACE2 und ACE2 mit Ang II stimuliert und bei 37°C für 16 Stunden inkubiert. Die Überstände wurden auf TNF-alpha untersucht und mit einem Kontrollansatz, der in Abwesenheit von ACE2 und Peptiden des RAS durchgeführt wurde, verglichen. Die Resultate dieses Versuches sind graphisch in Fig. 3 dargestellt: Die Inkubation mit LPS und PHA induzierte in allen Fällen die Sekretion von TNF-alpha. Die jeweiligen Kontrollansätze, die ohne ACE2 koinkubiert wurden, zeigten die höchsten TNF-alpha Konzentrationen (203, 352 und 278 mOD) jeweils nach LPS, PHA und der Kombinationsstimulation. In Gegenwart von ACE2 war das gemessene Signal in allen Gruppen deutlich geringer und erreichte nur noch mOD Werte von 181, 266, 223 in den jeweiligen Gruppen. Unter Anwesenheit von ACE2 und Ang II allerdings waren die gemessenen TNF-alpha Konzentrationen die geringsten und erreichten nur noch mOD 144, 247 und 183. Diese Ergebnisse zeigen auf, dass die Anwesenheit von ACE2 zu einer deutlich abgeschwächten Produktion inflammatorischer Zytokine führt, selbst wenn zur Stimulation besonders immunogene Substanzen wie LPS oder PHA verwendet werden. Dies bestätigt einen anti-inflammatorischen Effekt von ACE2. Erstaunlicherweise funktioniert der Mechanismus bereits ohne Anwesenheit von Ang II und wird in dessen Gegenwart verstärkt, was auf ein duales Prinzip hindeutet. Ein Teil des Effekts wird durch Ang II und dessen Abbauprodukt Ang 1-7 bewirkt, wobei ein weiterer Teil offensichtlich über den Abbau eines der anderen ACE2 Substrate funktioniert und nicht an vorhandenes Ang II gebunden ist (Fig. 3).

### Beispiel 3: Wiederherstellung der Ang II Titer des gesunden Organismus

In diesem Beispiel wurde aufgezeigt wie exogene ACE2 Gabe ein dereguliertes RAS wieder unter Kontrolle bringt. APN 01 (rekombinantes lösliches humanes ACE2) wurde hierfür in einem durch LPS Gabe induzierten Sepsis Modell verabreicht. Den Tieren wurde ab dem Zeitpunkt -120 Minuten kontinuierlich LPS infundiert, was zu einer massiven Inflammation und in weiterer Folge zu einer Sepsis führte. Es kam aufgrund der massiven Ausschüttung inflammtorischer Zytokine zum Abschalten der ACE2 Expression, was in weiterer Folge zu einer Akkumulation des inflammatorischen Peptides Ang II führte (siehe Fig. 4).

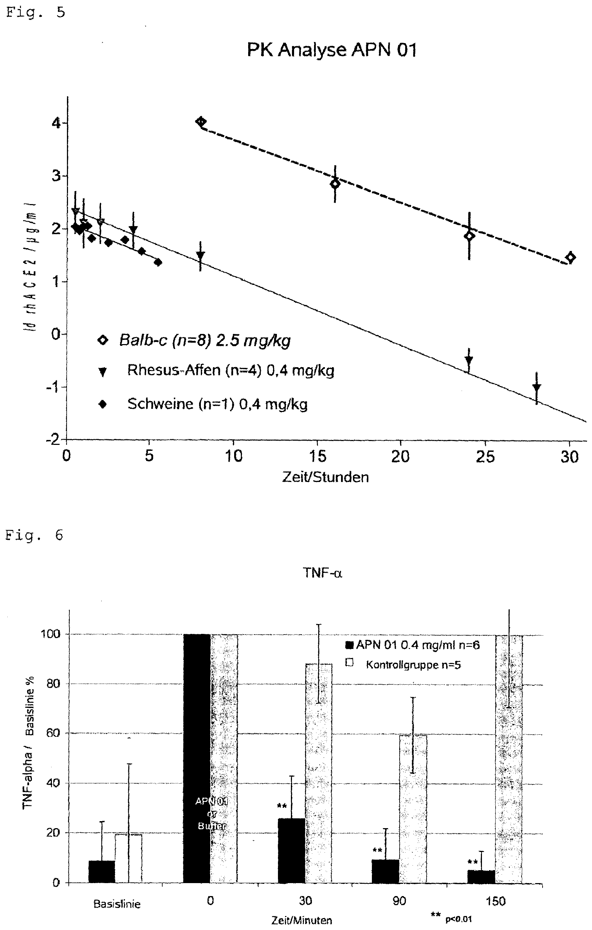

Ab dem Zeitpunkt 0 Minuten wurde APN 01 in einer Dosierung von 400 µg/kg als Bolus intra-venös verabreicht. Es kam sofort zu einer Abnahme von Ang II in der behandelten Gruppe, und die Ang II Titer pendelten sich binnen der darauf folgenden Stunde auf dasselbe Niveau, das auch in gesunden Tieren gemessen wurde, wieder ein. Ferner erbrachte die APN 01 Gabe derselben Dosis in gesunden Tieren ebenfalls einen kurzen Abfall der Ang II Titer, welche sich ebenfalls nach einer weiteren Stunde wieder den Werten der gesunden Tiere annäherten. Placebo behandelte Tiere zeigten hingegen einen weiter ansteigenden Ang II Wert bis zum Ende des Experimentes auf. Dieses erstaunliche Phänomen kann nur durch eine Wiederherstellung des hochregulierten RAS erklärt werden, da bis zum Ende des Experimentes nach wie vor aktives Enzym den Tieren systemisch zur Verfügung stand (siehe Fig. 5). Es wurde eine Halbwertszeit von ca. 8 Stunden gemessen.

### Beispiel 4: Attenuierung der Expression inflammatorischer Zytokine in der Sepsis

Im folgenden Beispiel wird gezeigt, wie die Konzentration des inflammatorischen Zytokins in einem Sepsismodell in Schweinen rapide zunimmt und nach ACE2 Gabe wieder auf das Niveau der gesunden Tiere zurückfällt. Den Tieren wurde ab dem Zeitpunkt -120 Minuten kontinuierlich LPS in hoher Dosierung infundiert, was zu einer massiven Inflammation und in weiterer Folge zu einer Sepsis führte. Es kam aufgrund der massiven Ausschüttung inflammtorischer Zytokine zum Abschalten der ACE2 Expression, was in weiterer Folge nicht nur zu einer Akkumulation des inflammatorischen Peptides Ang II, sondern ebenfalls des inflammatorischen Zytokins TNF-alpha führte (Fig. 6). Ab dem Zeitpunkt 0 Minuten wurde den Tieren (6 Tiere in der behandelten Gruppe, 5 Tiere in der Kontrollgruppe) entweder ACE2 in einer Dosierung von 0.4 mg/kg oder Pufferlösung als Bolus intravenös verabreicht. Während LPS weiters in derselben, hohen Dosierung kontinuierlich verabreicht wurde, wurden die Tiere für weitere 3 Stunden beobachtet und Serumproben gewonnen und auf TNF-alpha analysiert. Es konnte aufgezeigt werden, dass die TNF-alpha Konzentration in der Kontrollgruppe bis zum Ende des Experimentes erhöht blieb, während es in der ACE2 behandelten Gruppe bereits nach einmaliger ACE2 Verabreichung und bei fortwährender LPS Gabe zu einer deutlichen (p<0,001) Herabsetzung der TNF-alpha Konzentration kam. Es wurden, trotz massiver Sepsis, wieder in etwa dieselben Werte erreicht, die auch in gesunden Tieren gemessen wurden. Durch ACE2 Gabe konnte daher sogar in einem sehr aggressiven Sepsismodell die TNF-alpha Expression rapide auf das Niveau Gesunder herabgesetzt werden, und einer sich weiterhin potenzierenden Inflammation Einhalt geboten werden (Fig. 6).

### Beispiel 5: Attenuierung der Expression sämtlicher inflammatorischer Zytokine nach lokaler mechanischer Lungenschädigung

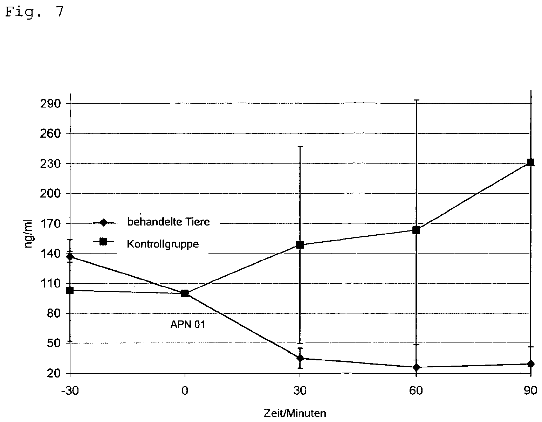

In diesem Beispiel wurde der Einfluss von systemisch verabreichtem ACE2 auf die Expression inflammatorischer Zytokine in einem Lungenschädigungsmodell in Schweinen aufgezeigt. 14 Tiere wurden in dieser Placebo kontrollierten, verblindeten Studie berücksichtigt. Alle wurden in der ersten Phase des Experimentes einer dreimaligen Aspiration einer 20% Mekoniumlösung unterzogen, wobei aufgrund erhobener hämodynamischer Parameter eine vergleichbare Schädigung in allen Tieren induziert wurde. In der zweiten Phase des Experimentes, der therapeutischen, wurde der einen Hälfte der Tiere rekombinantes lösliches humanes ACE2 intravenös als Bolus in einer Dosierung von 0,4 mg/kg verabreicht. Die andere erhielt eine physiologische Kochsalzlösung. Es wurden zu den Zeitpunkten -30, 0, 30, 60, 90 und 150 Minuten Serumproben abgenommen, in welchen die Konzentrationen der bedeutendsten inflammatorischen Zytokine gemessen wurden. Der Zeitpunkt 0 war hierbei der Startpunkt der Therapie, zu welchem alle Tiere bereits ARDS Symptome zeigten. Wie in der Fig. 7 verdeutlicht wird, gibt einen sehr deutlichen Einfluss der ACE2 Verabreichung auf die Serumkonzentration von TNF-alpha. Während diese in der Placebogruppe von über 230 ng/ml stark ansteigt, fällt sie in der behandelten Gruppe binnen 30 Minuten nach Verabreichung auf unter 40 ng/ml und nähert um 90 Minuten nach Gabe 25 ng/ml an.

### Beispiel 6: Diskussion

Die angeführte Daten lassen die folgenden Rückschlüsse auf die Wirkung von ACE2 als Immunregulator zu. Bedingt durch einen antigenen Stimulus kommt es zur Ausschüttung inflammatorischer Zytokine. In Gegenwart inflammatorischer Zytokine kommt es zu einem Verlust der ACE2 Expression. In Abwesenheit von ACE2 akkumuliert das pro-inflammatorische Peptid Ang II, da es nicht durch ACE2 abgebaut werden kann. In Abwesenheit von ACE2 akkumuliert ebenfalls das pro-inflammatorische Zytokin TNF-alpha. ACE2 besitzt anti-inflammatorische Eigenschaften und setzt in Lymphozyten die Expression inflammatorischer Zytokine herab. Eine therapeutische ACE2 Gabe kompensiert daher die verlorengegangene endogene ACE2 Expression und kann eine aufkeimende Inflammation durch Herabsetzung von Ang II Titern, durch Bildung von Ang 1-7 und durch andere Effekte abfangen. Eine therapeutische ACE2 Gabe ermöglicht es sogar im Falle einer schweren Sepsis unter kontinuierlicher LPS Infusion Ang II Titer wieder auf das Niveau eines Gesunden herabzusetzen und die Regulierung des RAS dementsprechend wieder herzustellen. Eine therapeutische ACE2 Gabe ermöglicht es ferner im Falle einer schweren Sepsis unter kontinuierlicher LPS Infusion TNF-alpha Titer wieder auf das Niveau des Gesunden herabzusetzen. Derselbe Effekt konnte ebenfalls im Falle einer mechanischen, massiven Lungenschädigung durch Mekoniumaspiration beobachtet werden.

## Patentansprüche

1. Rekombinantes ACE2 Protein oder eine Nukleinsäure, welche das rekombinante ACE2 Protein kodiert, zur Verwendung in der therapeutischen Behandlung oder Vorbeugung einer Inflammation wie Rheumatitis, Sepsis, Arthritis, rheumatoider Systemischer Lupus Erythematodes und Sklerodermie.

2. ACE2 Protein oder ACE2 kodierende Nukleinsäure zur Verwendung nach Anspruch 1, **dadurch gekennzeichnet, dass** die Inflammation durch eine Infektion verursacht ist.

3. ACE2 Protein oder ACE2 kodierende Nukleinsäure zur Verwendung nach Anspruch 1 oder 2, **dadurch gekennzeichnet, dass** das Protein rekombinantes ACE2 ist.

4. ACE2 Protein oder ACE2 kodierende Nukleinsäure zur Verwendung nach einem der Ansprüche 1 bis 3, **dadurch gekennzeichnet, dass** das Protein wasserlösliches ACE2, insbesondere ohne Membrandomäne, ist.

5. ACE2 Protein oder ACE2 kodierende Nukleinsäure zur Verwendung nach einem der Ansprüche 1 bis 4, **dadurch gekennzeichnet, dass** das Protein ACE2 von einem Säuger, vorzugsweise einem Menschen, Maus, Ratte, Hamster, Schwein, Primaten oder Rind ist.

6. Verwendung von rekombinantem ACE2 Protein oder einer Nukleinsäure, welche das rekombinante ACE2 Protein kodiert, zur Herstellung einer pharmazeutischen Zusammensetzung zur Behandlung einer Inflammation wie Rheumatitis, Sepsis, Arthritis, rheumatoider Systemischer Lupus Erythematodes und Sklerodermie.

## Claims

1. Recombinant ACE2 protein or a nucleic acid, which encodes the recombinant ACE2 protein, for use in the therapeutic treatment or prevention of an inflammation such as rheumatitis, sepsis, arthritis, rheumatoid systemic lupus erythematosus and scleroderma.

2. The ACE2 protein or ACE2-encoding nucleic acid for use according to claim 1, **characterized in that** the inflammation is caused by an infection.

3. The ACE2 protein or ACE2-encoding nucleic acid for use according to claim 1 or 2, **characterized in that** the protein is a recombinant ACE2.

4. The ACE2 protein or ACE2-encoding nucleic acid for use according to any one of claims 1 to 3, **characterized in that** the protein is a watersoluble ACE2, in particular without membrane domain.

5. The ACE2 protein or ACE2-encoding nucleic acid for use according to any one of claims 1 to 4, **characterized in that** the protein ACE2 originates from a mammal, preferably a human being, a mouse, a rat, a hamster, a pig, a primate or cattle.

6. Use of recombinant ACE2 protein or an ACE2-encoding nucleic acid, which encodes the recombinant ACE2 protein, for the production of a pharmaceutical composition for the treatment of an inflammation such as rheumatitis, sepsis, arthritis, rheumatoid systemic lupus erythematosus and scleroderma.

## Revendications

1. Protéine ACE2 recombinante ou acide nucléique qui code pour la protéine ACE2 recombinante, pour une utilisation dans le traitement thérapeutique ou la prévention d'une inflammation telle que le rhumatisme inflammatoire aigu, la sepsie, l'arthrite, le lupus érythémateux disséminé rhumatoïde et la sclérodermie.

2. Protéine ACE2 ou acide nucléique codant pour ACE2 pour l'utilisation selon la revendication 1, **caractérisé en ce que** l'inflammation est provoquée par une infection.

3. Protéine ACE2 ou acide nucléique codant pour ACE2 pour l'utilisation selon la revendication 1 ou 2, **caractérisé en ce que** la protéine est une protéine ACE2 recombinante.

4. Protéine ACE2 ou acide nucléique codant pour ACE2 pour l'utilisation selon l'une des revendications 1 à 3, **caractérisé en ce que** la protéine est une protéine ACE2 soluble dans l'eau, en particulier sans domaine membranaire.

5. Protéine ACE2 ou acide nucléique codant pour ACE2 pour l'utilisation selon l'une des revendications 1 à 4, **caractérisé en ce que** la protéine ACE2 est celle d'un mammifère, de préférence d'un humain, d'une souris, d'un rat, d'un hamster, d'un porc, d'un primate ou d'un bovin.

6. Utilisation d'une protéine ACE2 recombinante ou d'un acide nucléique qui code pour la protéine ACE2 recombinante, pour la fabrication d'une composition pharmaceutique destinée au traitement d'une inflammation, telle que le rhumatisme inflammatoire aigu, la sepsie, l'arthrite, le lupus érythémateux disséminé rhumatoïde et la sclérodermie.


### SEQUENCE LISTING

<110> Apeiron Biologics Forschungs- und Entwicklungsgesellschaft m.b.H. et al.
<120> Behandlung inflammatorischer Krankheiten
<130> r53546
<150> A 2058/2007
   <151> 2007 12 18
<160> 1
<170> PatentIn version 3.4
<210> 1
   <211> 740
   <212> PRT
   <213> homo sapiens
<400> 1
   Met Ser Ser Ser Ser Trp Leu Leu Leu Ser Leu Val Ala Val Thr Ala
   1               5                   10                  15
   Ala Gln Ser Thr Ile Glu Glu Gln Ala Lys Thr Phe Leu Asp Lys Phe
               20                  25                  30
   Asn His Glu Ala Glu Asp Leu Phe Tyr Gln Ser Ser Leu Ala Ser Trp
           35                  40                  45
   Asn Tyr Asn Thr Asn Ile Thr Glu Glu Asn Val Gln Asn Met Asn Asn
       50                  55                  60
   Ala Gly Asp Lys Trp Ser Ala Phe Leu Lys Glu Gln Ser Thr Leu Ala
   65                  70                  75                  80
   Gln Met Tyr Pro Leu Gln Glu Ile Gln Asn Leu Thr Val Lys Leu Gln
                   85                  90                  95
   Leu Gln Ala Leu Gln Gln Asn Gly Ser Ser Val Leu Ser Glu Asp Lys
               100                 105                 110
   Ser Lys Arg Leu Asn Thr Ile Leu Asn Thr Met Ser Thr Ile Tyr Ser
           115                 120                 125
   Thr Gly Lys Val Cys Asn Pro Asp Asn Pro Gln Glu Cys Leu Leu Leu
       130                 135                 140
   Glu Pro Gly Leu Asn Glu Ile Met Ala Asn Ser Leu Asp Tyr Asn Glu
   145                 150                 155                 160
   Arg Leu Trp Ala Trp Glu Ser Trp Arg Ser Glu Val Gly Lys Gln Leu
                   165                 170                 175
   Arg Pro Leu Tyr Glu Glu Tyr Val Val Leu Lys Asn Glu Met Ala Arg
               180                 185                 190
   Ala Asn His Tyr Glu Asp Tyr Gly Asp Tyr Trp Arg Gly Asp Tyr Glu
   195 200 205
   Val Asn Gly Val Asp Gly Tyr Asp Tyr Ser Arg Gly Gln Leu Ile Glu
   210 215 220
   Asp Val Glu His Thr Phe Glu Glu Ile Lys Pro Leu Tyr Glu His Leu
   225 230 235 240
   His Ala Tyr Val Arg Ala Lys Leu Met Asn Ala Tyr Pro Ser Tyr Ile
   245 250 255
   Ser Pro Ile Gly Cys Leu Pro Ala His Leu Leu Gly Asp Met Trp Gly
   260 265 270
   Arg Phe Trp Thr Asn Leu Tyr Ser Leu Thr Val Pro Phe Gly Gln Lys
   275 280 285
   Pro Asn Ile Asp Val Thr Asp Ala Met Val Asp Gln Ala Trp Asp Ala
   290 295 300
   Gln Arg Ile Phe Lys Glu Ala Glu Lys Phe Phe Val Ser Val Gly Leu
   305 310 315 320
   Pro Asn Met Thr Gln Gly Phe Trp Glu Asn Ser Met Leu Thr Asp Pro
   325 330 335
   Gly Asn Val Gln Lys Ala Val Cys His Pro Thr Ala Trp Asp Leu Gly
   340 345 350
   Lys Gly Asp Phe Arg Ile Leu Met Cys Thr Lys Val Thr Met Asp Asp
   355 360 365
   Phe Leu Thr Ala His His Glu Met Gly His Ile Gln Tyr Asp Met Ala
   370 375 380
   Tyr Ala Ala Gln Pro Phe Leu Leu Arg Asn Gly Ala Asn Glu Gly Phe
   385 390 395 400
   His Glu Ala Val Gly Glu Ile Met Ser Leu Ser Ala Ala Thr Pro Lys
   405 410 415
   His Leu Lys Ser Ile Gly Leu Leu Ser Pro Asp Phe Gln Glu Asp Asn
   420 425 430
   Glu Thr Glu Ile Asn Phe Leu Leu Lys Gln Ala Leu Thr Ile Val Gly
   435 440 445
   Thr Leu Pro Phe Thr Tyr Met Leu Glu Lys Trp Arg Trp Met Val Phe
   450 455 460
   Lys Gly Glu Ile Pro Lys Asp Gln Trp Met Lys Lys Trp Trp Glu Met
   465 470 475 480
   Lys Arg Glu Ile Val Gly Val Val Glu Pro Val Pro His Asp Glu Thr
   485 490 495
   Tyr Cys Asp Pro Ala Ser Leu Phe His Val Ser Asn Asp Tyr Ser Phe
   500 505 510
   Ile Arg Tyr Tyr Thr Arg Thr Leu Tyr Gln Phe Gln Phe Gln Glu Ala
   515 520 525
   Leu Cys Gln Ala Ala Lys His Glu Gly Pro Leu His Lys Cys Asp Ile
   530 535 540
   Ser Asn Ser Thr Glu Ala Gly Gln Lys Leu Phe Asn Met Leu Arg Leu
   545 550 555 560
   Gly Lys Ser Glu Pro Trp Thr Leu Ala Leu Glu Asn Val Val Gly Ala
   565 570 575
   Lys Asn Met Asn Val Arg Pro Leu Leu Asn Tyr Phe Glu Pro Leu Phe
   580 585 590
   Thr Trp Leu Lys Asp Gln Asn Lys Asn Ser Phe Val Gly Trp Ser Thr
   595 600 605
   Asp Trp Ser Pro Tyr Ala Asp Gln Ser Ile Lys Val Arg Ile Ser Leu
   610 615 620
   Lys Ser Ala Leu Gly Asp Lys Ala Tyr Glu Trp Asn Asp Asn Glu Met
   625 630 635 640
   Tyr Leu Phe Arg Ser Ser Val Ala Tyr Ala Met Arg Gln Tyr Phe Leu
   645 650 655
   Lys Val Lys Asn Gln Met Ile Leu Phe Gly Glu Glu Asp Val Arg Val
   660 665 670
   Ala Asn Leu Lys Pro Arg Ile Ser Phe Asn Phe Phe Val Thr Ala Pro
   675 680 685
   Lys Asn Val Ser Asp Ile Ile Pro Arg Thr Glu Val Glu Lys Ala Ile
   690 695 700
   Arg Met Ser Arg Ser Arg Ile Asn Asp Ala Phe Arg Leu Asn Asp Asn
   705 710 715 720
   Ser Leu Glu Phe Leu Gly Ile Gln Pro Thr Leu Gly Pro Pro Asn Gln
   725 730 735
   Pro Pro Val Ser
   740